(19) Europäisches Patentamt
European Patent Office
Office européen des brevets

(11) **EP 4 509 826 A1**

(12) **EUROPEAN PATENT APPLICATION**
published in accordance with Art. 153(4) EPC

(43) Date of publication:
**19.02.2025 Bulletin 2025/08**

(21) Application number: **23788116.4**

(22) Date of filing: **17.03.2023**

(51) International Patent Classification (IPC):
***G01N 27/416*** (2006.01)  ***G01N 27/30*** (2006.01)
***G01N 35/08*** (2006.01)  ***G01N 37/00*** (2006.01)

(52) Cooperative Patent Classification (CPC):
**G01N 27/30; G01N 27/416; G01N 35/08;
G01N 37/00**

(86) International application number:
**PCT/JP2023/010501**

(87) International publication number:
**WO 2023/199694 (19.10.2023 Gazette 2023/42)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **12.04.2022 JP 2022065895**

(71) Applicant: **CANON KABUSHIKI KAISHA
Tokyo 146-8501 (JP)**

(72) Inventors:
• **YAMAMOTO, Takeshi
Tokyo 146-8501 (JP)**

• **MIYAZAKI, Keiji
Tokyo 146-8501 (JP)**
• **MIURA, Jun
Tokyo 146-8501 (JP)**
• **MAEDA, Harunobu
Tokyo 146-8501 (JP)**
• **ENOKIDO, Fuka
Tokyo 146-8501 (JP)**
• **TANAKA, Masanori
Tokyo 146-8501 (JP)**
• **FUKATSU, Makoto
Tokyo 146-8501 (JP)**

(74) Representative: **TBK
Bavariaring 4-6
80336 München (DE)**

(54) **MICROANALYSIS CHIP**

(57) A micro analysis chip capable of performing stable analysis is provided. The micro analysis chip includes a flow passage region surrounded by a flow passage wall disposed in the interior of a porous substrate, wherein the flow passage region includes a first flow passage chamber, a second flow passage chamber, and a flow passage to connect the first flow passage chamber to the second flow passage chamber, a reference electrode is disposed in the first flow passage chamber, a working electrode is disposed in the second flow passage chamber, and an electrolyte is arranged upstream of the reference electrode with reference to a forward direction of a specimen in the flow passage region.

FIG. 5A

EP 4 509 826 A1

**Description**

Technical Field

The present invention relates to a micro analysis chip in which a micro flow passage is formed in the interior of a porous substrate, an electrolyte concentration measuring system including the micro analysis chip, and an electrolyte concentration measuring method.

Background Art

In recent years, development of a micro flow passage device capable of efficiently (very small amount, rapid, and simple) performing biochemical analysis in a chip by utilizing a micro-sized, fine flow passage has attracted attention in wide fields of biochemical research, medicine, drug discovery, health care, environment, food, and the like.

In particular, a paper micro analysis chip based on paper uses an inexpensive material such as paper as a substrate, enables a specimen or an inspection liquid to be driven by utilizing a capillary phenomenon of paper itself without using an electric power, and, therefore, enables any one to readily realize inspection and diagnosis based on POC (point of care) at low cost due to a small size, a low cost, ease of carrying, high disposability (disposal is completed by only incineration), and no need for a large apparatus. Consequently, there are global expectations as an inspection device in medical services in developing countries and remote areas where medical equipment is not sufficiently prepared, at disaster sites, and the like, and at air terminals and the like where infections must be prevented from spreading at the water's edge. In addition, attention is attracted as a health care device capable of controlling and monitoring the health state by oneself and various pathological diagnosis devices in usual medical sites.

Electrolyte measurement is one of biochemical inspections of the pathological diagnosis. The electrolyte measurement is an inspection for measuring an ion concentration ($Na^+$, $K^+$, $Cl^-$, or the like) in blood or in urine. That is, the electrolyte measurement is an inspection to measure "ionic concentration balance in a body". The ionic concentration in a body reflects the amount of water and the pH in a body, and to maintain life by normally functioning transmission through nerves, a heart, and muscles, it is important that the ionic concentration is appropriately controlled.

In this regard, for example, when a change occurs in the electrolyte concentration, there is a high likelihood that an irregularity occurred in kidney functions or hormone effects. Therefore, the electrolyte measurement is an indispensable inspection for screening disease and, further, is a very important inspection for examining the physiological functions (life state) of a patient in a disaster site or the like.

Various universities and companies perform research on the electrolyte measurement by using μPADs.

NPL 1 proposes a filter-paper-based measuring device for the concentration of a Na ion and a K ion. This device includes a dispensation portion to dispense a specimen, and a dispensed specimen permeating from the dispensation portion into the respective regions of a working electrode and a reference electrode electrically couples the two electrodes to each other so as to enable the potentiometry to be performed. In this regard, in the above-described device, to obtain a stable potential at the reference electrode, a KCl ion crystal is accumulated on the reference electrode, and KCl dissolving in the specimen during measurement maintains a high Cl ion concentration in the reference electrode region and obtains a stable potential of the reference electrode. Further, only a measurement target ion being selected by an ion-selective film formed covering the working electrode enables the measurement to be performed without being affected by other ions.

In addition, PTL 1 discloses a method in which a stable potential is obtained at the reference electrode by dispensing a specimen in a working electrode portion and simultaneously dispensing a saturated solution of KCl or NaCl serving as a standard solution in a reference electrode portion.

Citation List

Patent Literature

PTL 1: Japanese Patent Laid-Open No. 62-039758

Non Patent Literature

NPL 1: Nipapan Ruecha, Orawon Chailapakul, Koji Suzuki and Daniel Chitterio, "Fully Inkjet-Printed Paper-Based Potentiometric Ion-Sensing Devices", Analytical chemistry, August 29, 2017 Published, 89, pp. 10608-10616

Summary of Invention

Technical Problem

However, arrangement of an electrolyte layer (KCl layer) is very difficult, and the potential of the reference electrode is not stabilized by only simply stacking and arranging the KCL layer on a support electrolyte or AgCl. Consequently, even when the concentration of the specimen is the same, variations between devices increase, and it is difficult to obtain stable measurement results. This problem was found on the basis of the investigation by the present inventors.

It is an object of the present invention to provide a micro analysis chip in which an electrolyte layer is arranged at an appropriate position, which indicates an appropriate potential of a reference electrode, and which is capable of performing stable analysis.

Solution to Problem

The present invention relates to a micro analysis chip including a flow passage region surrounded by a flow passage wall disposed in the interior of a porous substrate,

wherein the flow passage region includes a first flow passage chamber, a second flow passage chamber, and a flow passage to connect the first flow passage chamber to the second flow passage chamber,
a reference electrode is disposed in the first flow passage chamber,
a working electrode is disposed in the second flow passage chamber, and
an electrolyte is arranged upstream of the reference electrode with reference to a forward direction of a specimen in the flow passage region.

Advantageous Effects of Invention

According to the present invention, a micro analysis chip capable of performing stable analysis is provided.

Brief Description of Drawings

[Fig. 1A] Fig. 1A is a configuration diagram illustrating a reference electrode.
[Fig. 1B] Fig. 1B is a configuration diagram illustrating a flow passage pattern.
[Fig. 1C] Fig. 1C is a sectional view of a reference electrode portion.
[Fig. 2A] is a schematic diagram illustrating potentiometric titration.
[Fig. 2B] Fig. 2B is a graph illustrating the Cl⁻concentration versus the potential change.
[Fig. 3] Fig. 3 is an explanatory diagram illustrating the Cl⁻ concentration versus the potential change of the reference electrode.
[Fig. 4] Fig. 4 is a graph illustrating variations in the potential of the reference electrode.
[Fig. 5A] Fig. 5A is a configuration diagram of the reference electrode and the working electrode.
[Fig. 5B] Fig. 5B is a graph illustrating the Na⁺ concentration versus the potential change.
[Fig. 6] Fig. 6 is a graph illustrating the Na⁺ concentration versus the potential change between the reference electrode and the working electrode.
[Fig. 7A] Fig. 7A is an explanatory diagram illustrating the configuration of an example in the related art.
[Fig. 7B] Fig. 7B is an explanatory diagram illustrating the configuration of an example in the related art.
[Fig. 8A] Fig. 8A is an explanatory diagram illustrating a phenomenon due to the configuration of an example in the related art.
[Fig. 8B] Fig. 8B is an explanatory diagram illustrating a phenomenon due to the configuration of an example in the related art.
[Fig. 8C] Fig. 8C is a graph illustrating variations in the potential of the reference electrode.
[Fig. 9A] Fig. 9A is an explanatory diagram illustrating the configuration of an example in the related art.
[Fig. 9B] Fig. 9B is an explanatory diagram illustrating the configuration of an example in the related art.
[Fig. 10] Fig. 10 is a sectional view illustrating an analysis chip including a laminate layer.
[Fig. 11A] Fig. 11A is a configuration diagram illustrating the reference electrode including an ion-selective film on the surface.
[Fig. 11B] Fig. 11B is a graph illustrating the Na⁺ concentration versus the potential change.

[Fig. 12A] Fig. 12A is a configuration diagram illustrating another reference electrode.
[Fig. 12B] Fig. 12B is a configuration diagram illustrating another reference electrode.

Description of Embodiments

The operation of the present invention will be described in detail.

The role of the reference electrode is to serve as a reference potential relative to a potential generated at the working electrode. Therefore, the reference electrode has to always indicate the same potential relative to any concentration of specimen. The potential between the reference electrode and the working electrode is incorrect unless the potential of the reference electrode is stable.

In general, Ag/AgCl is frequently used as the base electrode of the reference electrode. Regarding Ag/AgCl, the following equilibrium reaction occurs at an interface to the specimen, and the potential is determined in accordance with a Cl⁻ concentration.

[Math. 1]

$$Ag + Cl^- \longleftrightarrow AgCl + e^-$$

Consequently, the Cl⁻ concentration being constant enables the potential to be stabilized.

Since a Cl⁻ concentration is constant in a saturated sodium chloride (NaCl) solution or a saturated potassium chloride (KCl) solution, the same interfacial potential is always obtained by reacting the saturated NaCl solution or the saturated KCl solution with AgCl.

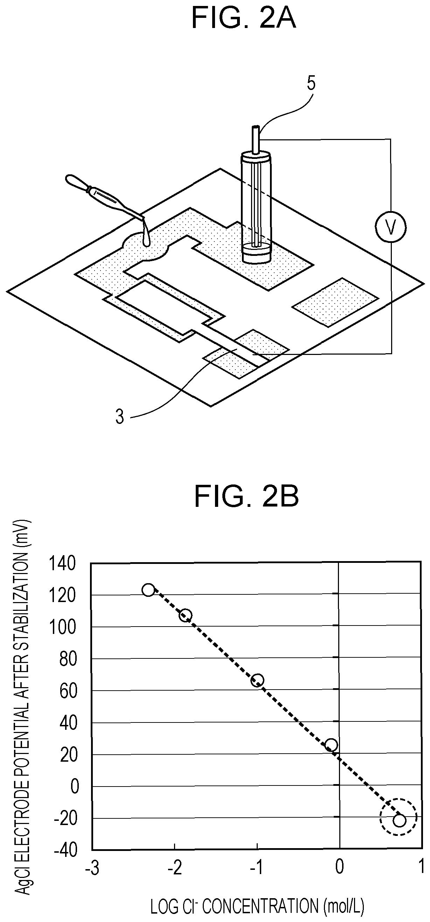

Fig. 2B illustrates the results of potential measurement when a NaCl solution was directly dispensed in a Ag/AgCl electrode 3 by using a flow passage of a micro analysis chip while changing the concentration of NaCl (Cl⁻ concentration). Regarding the measurement, as illustrated in Fig. 2A, a commercially available electrode 5 is placed on one side, the Ag/AgCl electrode 3 is connected to the commercially available electrode 5 through the NaCl solution, a potential between the electrodes in such an instance was measured, and the potential change in accordance with the concentration change of NaCl was plotted.

When the NaCl concentration, that is, the Cl⁻ concentration, is increased, the potential of the Ag/AgCl electrode is decreased. When the NaCl concentration reaches a concentration close to saturation (about 5.2 mol/L when a temperature is 25°C), since NaCl does not dissolve any more, the Cl⁻ concentration becomes constant, the above-described equilibrium reaction is stopped, and the potential is not changed any more. In this regard, since it is conjectured that the measurement is frequently performed at room temperature (about 25°C), in the following explanation, use at room temperature (about 25°C) is assumed, and the design is based on the saturated concentration at 25°C. An amount of an electrolyte arranged in Example described later is an amount on the assumption of 25°C. However, when use in a different environment is assumed, it is sufficient that the design is performed in accordance with the use environment, and the configuration is not limited to the configuration (amount of electrolyte) on the assumption of 25°C.

Based on the above-described principle, in general, regarding an internal-liquid-type reference electrode, saturated KCl or NaCl is used as an internal liquid, and regarding a solid-electrode type, a technique to ensure a stabilized potential of a reference electrode by directly dispensing the saturated liquid serving as a standard liquid in a Ag/AgCl electrode is adopted.

According to the above, however, a reference liquid has to be prepared separately from a specimen, and regarding a micro analysis chip that is a simple, low-cost inspection device, there is a time and effort issue and a cost issue.

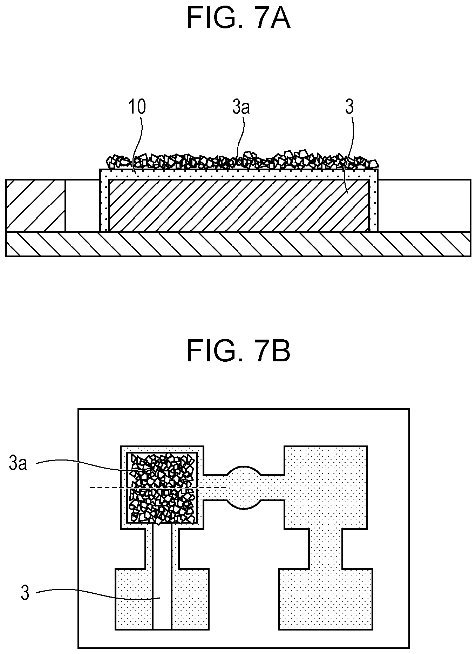

To address the above-described issues, NPL 1 describes a configuration in which, as illustrated in Fig. 7A and Fig. 7B, an electrolyte layer 3a is stacked on a support electrolyte film (disposed to further stabilize a contact potential at an interface between a specimen and a Ag/AgCl electrode) 9 disposed on a reference electrode (Ag/AgCl electrode) 3, the electrolyte layer is dissolved by the specimen, and a saturated concentration of the specimen is supplied to the support electrolyte film and the Ag/AgCl electrode so as to stabilize an interfacial potential.

However, even when the electrolyte is stacked on the Ag/AgCl electrode or the support electrolyte film by using various printing techniques, such as ink jet and dispenser, and adopting various electrolyte concentrations and pitch intervals, it is difficult to obtain a stable potential.

A test to ascertain that it is difficult to obtain a stable potential by the above-described configuration was performed as described below.

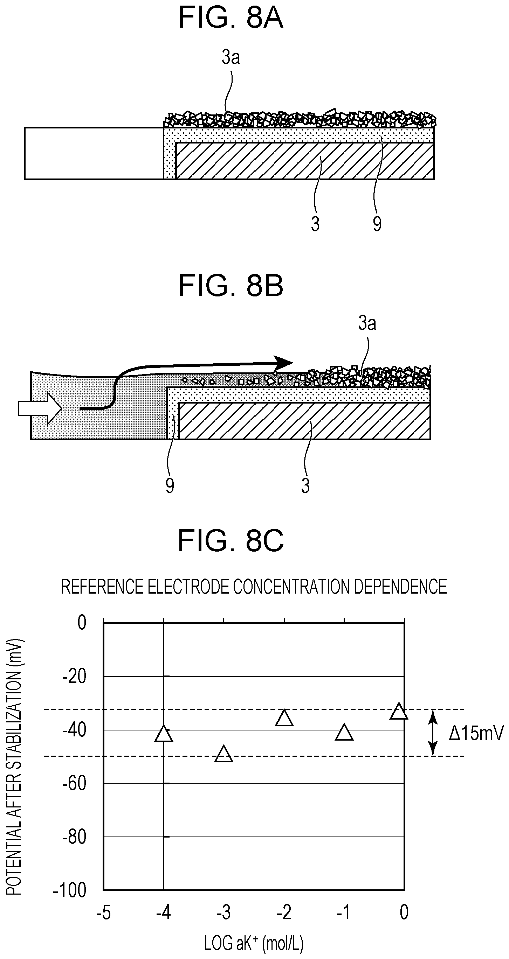

A sheet including a first flow passage chamber, a second flow passage chamber, and a flow passage to connect the first flow passage chamber to the second flow passage chamber was prepared. The reference electrode (Ag/AgCl electrode) 3 was arranged in the first flow passage chamber of the sheet, the support electrolyte film 9 was stacked on the electrode, further, the electrolyte layer (KCl layer) 3a was stacked on the support electrolyte film 9, and a commercially available electrode 5 was disposed in the second flow passage chamber so as to form a micro analysis chip. Regarding the micro analysis chip, a KCl solution serving as a specimen was dripped to the flow passage portion connected to the Ag/AgCl electrode and the commercially available electrode, and a potential difference between the Ag/AgCl electrode and the reference electrode was measured where the concentration of the KCl solution was changed. The concentration of the KCl solution was changed in the range of $10^{-4}$ to 800 mmol/L. The results of the test are illustrated in Fig. 8C. Essentially, the potential of the Ag/AgCl electrode has to be constant even when the specimen concentration is changed. However, variations in the potential were close to 15 mV.

The reason for an occurrence of variations in the potential is considered to be as described below.

A specimen that reaches the electrolyte layer first dissolves the electrolyte and comes into contact with the Ag/AgCl electrode while the Cl⁻ concentration is saturated. But the specimen is swept away by a specimen that flows thereafter (Fig. 8A, Fig. 8B). In this regard, the specimen that flows thereafter comes into contact with the Ag/AgCl electrode while the Cl⁻ concentration is not saturated since the electrolyte has been dissolved and is not present (or swept away). Consequently, it is conjectured that since both the specimen having a saturated Cl⁻ concentration and the specimen having an unsaturated Cl⁻ concentration simultaneously come into contact with the Ag/AgCl electrode, the potential is unstable.

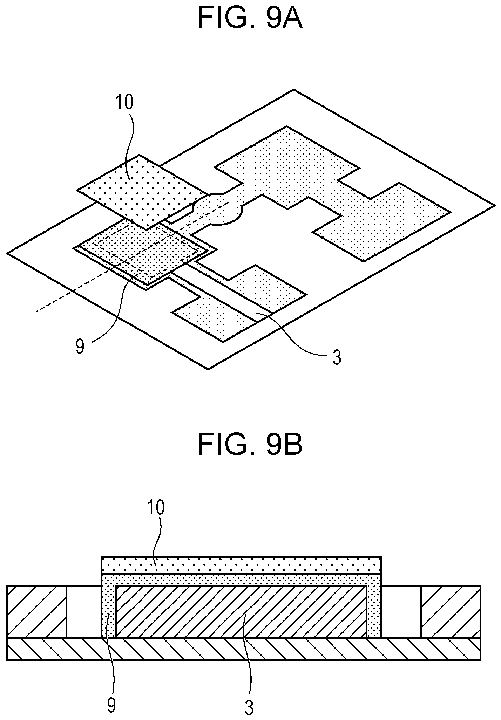

In addition, as illustrated in Fig. 9A and Fig. 9B, even when filter paper 10 impregnated with the electrolyte is arranged on the reference electrode (Ag/AgCl electrode) 3 or the support electrolyte film 9, the result was the same. Fig. 9B illustrates a cross section taken along a dotted line portion in Fig. 9A.

That is, it was ascertained that the measured interfacial potential is not stabilized unless a configuration in which the specimen having a saturated Cl⁻concentration is stably supplied to the Ag/AgCl electrode is established.

The micro analysis chip according to the present invention is characterized in that the electrolyte is arranged upstream of the reference electrode with reference to a forward direction of the specimen in the flow passage region, and the potential of the reference electrode is stabilized by the specimen coming into contact with the reference electrode while the Cl⁻ concentration is saturated.

That is, the micro analysis chip according to the present invention desirably has a configuration in which a predetermined amount of the electrolyte is arranged at a position so that an upstream end portion of the reference electrode is located downstream of a position where an electrolyte concentration in the specimen is a concentration close to saturation (88% of the saturated concentration) due to the electrolyte being dissolved in the specimen passing through the flow passage. In this regard, "88% of the saturated concentration" corresponds to about 4.6 mol/L with respect to a NaCl solution in which saturation is 5.2 mol/L.

Further, the micro analysis chip according to the present invention may have a configuration in which, for example, a third flow passage chamber provided with a working electrode is included in addition to the first flow passage chamber and the second flow passage chamber, these being connected by a flow passage. In this regard, there is no particular limitation regarding the number of flow passage chambers arranged on the micro analysis chip.

EXAMPLES

Exemplary embodiments according to the present invention will be described below with reference to the drawings. In this regard, the following embodiments are exemplifications, and the present invention is not limited to the contents of the embodiments. Regarding the following drawings, constituent elements unnecessary for explanation of the embodiments are omitted from the drawings.

<Example 1>

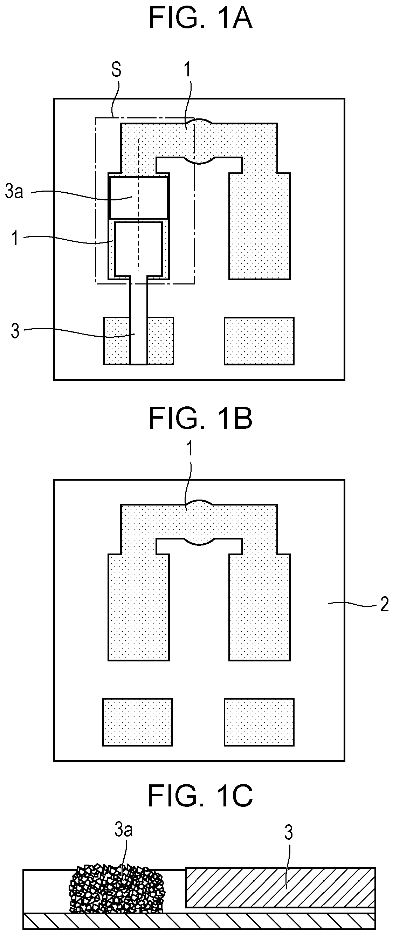

Example 1 will be described with reference to Fig. 1A, Fig. 1B, and Fig. 1C to Fig. 6.

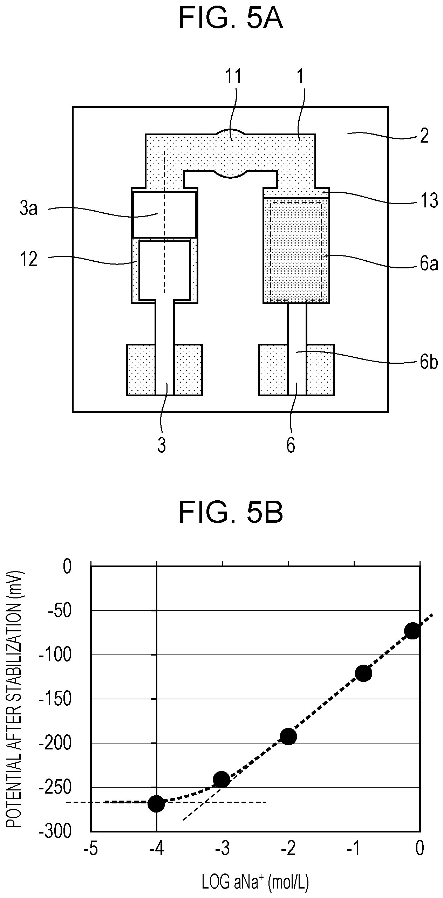

Fig. 5A illustrates a micro analysis chip of Example 1. A flow passage portion 1 surrounded by a flow passage wall 2 disposed in the interior of a porous substrate is included. The flow passage region 1 includes a first flow passage chamber 12, a second flow passage chamber 13, and a flow passage to connect the first flow passage chamber 12 to the second flow passage chamber 13, and a dispensation portion 11 is present in the flow passage. A reference electrode 3 is arranged in the first flow passage chamber 12, an electrolyte layer 3a is arranged upstream of the reference electrode 3 with reference to a forward direction of a specimen (that is, the dispensation portion 11 side is the upper stream). Since the electrolyte layer 3a is arranged upstream of the reference electrode 3, the specimen in the state in which the electrolyte concentration is saturated stably reaches the reference electrode. A working electrode 6 is arranged in the second flow passage chamber 13, and the working electrode 6 is composed of a base electrode 6b and an ion-selective film 6a disposed to cover the base electrode 6b.

The passage was formed by a method described in (Japanese Patent Laid-Open No. 2021-37612). Specifically, a flow passage forming particle (toner) characterized by meltability was used, an electrophotographic system was adopted, and a predetermined flow passage pattern in an unfixed state was formed on filter paper. Thereafter, the flow passage pattern was made to permeate the interior of the paper by an oven or a heater so as to form a flow passage pattern. The resulting flow passage pattern is as illustrated in Fig. 1B, reference 1 denotes "flow passage", and reference 2 denotes "flow passage wall".

Subsequently, the reference electrode and the working electrode were formed on the flow passage pattern by screen printing, an ink jet apparatus, a dispenser, or the like. Since the present invention relates to a reference electrode, the configuration is described focusing on the reference electrode.

As illustrated in Fig. 1A, a Ag/AgCl electrode serving as the reference electrode was printed on the flow passage pattern by screen printing. Further, as illustrated in Fig. 1A and Fig. 1C (Fig. 1C is a sectional view of a cross section taken along a dotted line in Fig. 1A), the electrolyte (NaCl or KCl) was printed by an ink jet apparatus or a dispenser at a position on the flow passage and upstream of the Ag/AgCl electrode with reference to a forward direction of the specimen. Due to the electrolyte layer 3a (NaCl or KCl) being arranged at such a position, the moving specimen surely passes through the electrolyte layer 3a. In such an instance, due the electrolyte being dissolved, the concentration of the electrolyte ($Cl^-$ concentration) in the specimen is saturated, and the specimen in such a state reaches the reference electrode (Ag/AgCl electrode). As a result, the potential of the reference electrode (Ag/AgCl electrode) is stabilized.

Regarding the electrolyte, a chloride is favorable, and in particular, sodium chloride (NaCl) or potassium chloride (KCl) which are easy to handle are favorable. Hereafter, explanations will be performed with reference to NaCl having solubility that exhibits more stable temperature dependence.

The electrolyte (NaCl) was arranged in layers on the flow passage before the reference electrode so that the amount was set to be $3.0 \times 10^2$ g/L or more and preferably $3.4 \times 10^2$ g/L or more per liter of the amount of the specimen supplied to the reference electrode. This is a value obtained by dividing the amount of NaCl necessary for saturation by the amount of the specimen supplied to the reference electrode since the saturated concentration of the electrolyte also depends on the amount of the specimen. When $3.0 \times 10^2$ g/L or more per liter of the amount of the specimen supplied to the reference electrode of NaCl is arranged, the concentration in the specimen passed through the NaCl layer is 4.6 mol/L or more and is close to the saturated concentration at 25°C so as to enable stable measurement to be performed. Preferably, $3.4 \times 10^2$ g/L or more of NaCl is arranged, and in such an instance, the concentration in the specimen passed through the NaCl layer is almost the saturated concentration (5.2 mol/L) and is a concentration sufficient for maintaining the state in which the $Cl^-$ concentration is almost saturated. There is no particular limitation regarding the upper limit, and $5.6 \times 10^2$ g/L or less is appropriate since NaCl may overflow during NaCl coating.

In this regard, when the electrolyte is KCl, it is sufficient that the amount of the electrolyte used is $3.0 \times 10^2$ g/L or more and preferably $3.4 \times 10^2$ g/L or more per liter of the amount of the specimen supplied to the reference electrode. In such an instance, the concentration in the specimen is 3.6 mol/L or more and 4.2 mol/L or more, respectively, and is a value of the saturated concentration of a KCl solution or close to this.

In this regard, there is an appropriate amount of specimen supplied to the dispensation portion in accordance with the size and the performance of each device, and in general, the amount is about 10 μL to 50 μL. For example, in the instance in which the size of the reference electrode is 3 mm × 3 mm, the size of the working electrode is 3 mm × 3 mm, and the thickness of the paper is 200 μm as an example of a small device, the volume of the total flow passage including the flow passage between these is about $3.6 \times 10^{-9}$ $m^3$ (3.6 μL) (reference electrode is 1.8 μL and working electrode is 1.8 μL). Consequently, the specimen being about 10 μL enables the specimen to be sufficiently supplied to the reference electrode and the working electrode.

When the size of the device increases, a necessary amount of the specimen increases. However, since the base of the specimen is human blood or urine, a small amount is favorable, and, in general, the amount is 50 μL or less. The present invention does not depend on the size of the device and the necessary amount of the specimen. However, for the above-described reason, the explanation will be provided on the assumption that the amount of the specimen is within the range of about 10 μL to 50 μL.

Regarding the micro analysis chip of Example 1, 10 μL of the specimen is dispensed and supplied to a dispensation portion 11 located at the center of the flow passage region, about a half thereof is supplied to the reference electrode 3 side (S portion in Fig. 1A), and the other half is supplied to the working electrode side. Therefore, the amount of the specimen supplied to the reference electrode 3 is about 5 μL. In this regard, in the present example, the electrolyte layer 3a was arranged so that the S portion in Fig. 1A had saturated concentration. In such an instance, when a total amount of the arranged electrolyte is dissolved, the electrolyte concentration in the specimen becomes a concentration 88% or more of the saturated concentration. Consequently, the concentration between the electrolyte layer 3a and the base electrode 3 in Fig. 1A to Fig. 1C is a concentration 88% or more of the saturated concentration. Therefore, the present configuration satisfies the configuration "the electrolyte is arranged at a position so that an upstream end portion of the reference electrode is located downstream of a position where an electrolyte concentration in a specimen is a concentration 88% or more of a saturated concentration".

For example, when the amount of the specimen supplied to the reference electrode is 5 μL, 4.6 mol/L or more of NaCl concentration is obtained by arranging 1.5 mg of NaCl. Consequently, when NaCl is arranged having a thickness of 200 $\mu$m in a 3 mm × 3 mm region, the NaCl concentration in the specimen passed through the NaCl layer becomes 4.6 mol/L or more that is close to saturation by arranging $8.3 \times 10^5$ g/m$^3$ or more of NaCl per unit volume of a NaCl arrangement region. Further, when $9.4 \times 10^5$ g/m$^3$ or more of NaCl per unit volume of the NaCl arrangement region is arranged, the concentration becomes close to the saturated concentration (about 5.2 mol/L) in the specimen passed through the NaCl layer and is a concentration sufficient for making the Ag/AgCl electrode into an equilibrium state. In this regard, to specify a concentration of a solution in which NaCl is dissolved in the specimen, the specific gravity of the solution has to be taken into consideration, and the above-described NaCl concentration is a value in consideration of it.

When the amount of the specimen supplied to the reference electrode is 25 $\mu$L, and NaCl is arranged having a thickness of 200 $\mu$m in a 3 mm × 3 mm region, as described above, the NaCl concentration in the specimen passed through the NaCl layer becomes 4.6 mol/L or more by arranging $4.2 \times 10^6$ g/m$^3$ or more of NaCl per unit volume of the NaCl arrangement region. To set the NaCl concentration in the specimen passed through the NaCl layer to be almost saturated concentration (about 5.2 mol/L), it is sufficient that $4.7 \times 10^6$ g/m$^3$ or more of NaCl per unit volume of the NaCl arrangement region is arranged.

In addition, when the amount of the specimen supplied to the reference electrode is 5 $\mu$L, and NaCl is arranged having a thickness of 200 $\mu$m in a 6.7 mm × 6.7 mm region, the NaCl concentration in the specimen passed through the NaCl layer becomes 4.6 mol/L or more by arranging $1.7 \times 10^5$ g/m$^3$ or more of NaCl per unit volume of the NaCl arrangement region. To set the NaCl concentration in the specimen passed through the NaCl layer to be almost saturated concentration (about 5.2 mol/L), it is sufficient that $1.9 \times 10^5$ g/m$^3$ or more of NaCl per unit volume of the NaCl arrangement region is arranged.

When the specimen reaches the arranged electrolyte (NaCl) layer 3a, the specimen moves in the direction of the reference electrode (Ag/AgCl electrode) 3 while dissolving the arranged electrolyte (NaCl). In such an instance, the Cl$^-$ concentration in the specimen passed through the electrolyte (NaCl) layer is always maintained in the state of saturation or close to saturation, and since the saturated specimen is continuously supplied to the reference electrode (Ag/AgCl electrode), a stable potential is obtained.

In the present configuration (Fig. 1A), the reference electrode 3 and the electrolyte layer 3a is not in contact with each other. However, the electrolyte layer 3a may be in contact with the reference electrode 3 or may be stacked on the reference electrode 3. An important point is that the specimen reaches the reference electrode 3 after the electrolyte concentration in the specimen is saturated.

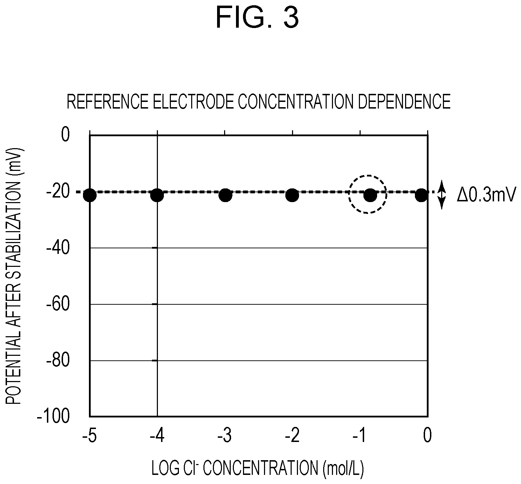

The reference electrode potential was measured using the specimen where the electrolyte (NaCl) concentration was changed from 10$^{-5}$ mol/L to 1 mol/L. The results are illustrated in Fig. 3. To pay attention to only the performance of the reference electrode, a commercially available electrode 4 in stead of a working electrode was placed on one side on which the working electrode is to be essentially formed in the micro analysis chip, the commercially available electrode was connected to the reference electrode through the specimen, and a potential difference between the two was measured, as in the instance of Fig. 2A and Fig. 2B.

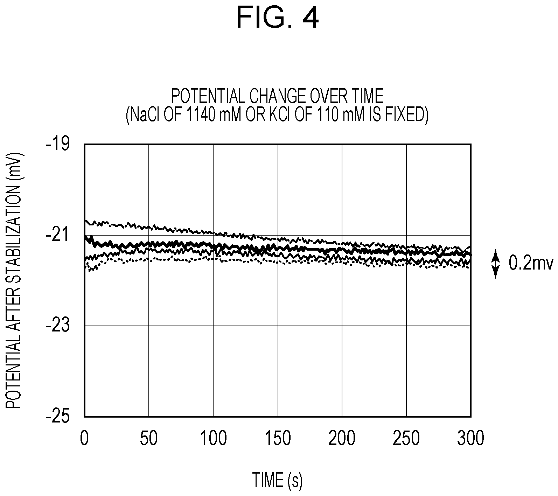

As a result of the measurement, it was ascertained that the potentials obtained by using specimens having different concentrations were constant. Variations when specimens having different concentrations were used were within about 0.3 mV (Fig. 3). In addition, a plurality of micro analysis chips formed in the same manner were used, and measurement was performed at the same concentration (140 mmol/L, dotted line circle in Fig. 3). It was ascertained that variations were within about 0.2 mV (Fig. 4).

Further, as illustrated in Fig. 5A, a working electrode serving as an ion-selective electrode was arranged on one side, the specimen was dispensed in the state in which both electrodes were arranged, and a potential between the working electrode and the reference electrode was measured. Regarding the specimen, a solution in which the electrolyte (Na$^+$) concentration was 10$^{-5}$ mol/L to 1 mol/L and to which an interfering ion was added (about 5 mmol/L of KCl when K$^+$ was adopted) was used. The results are illustrated in Fig. 5B.

Regarding the working electrode (ion-selective electrode), a solid-contact-type ion-selective electrode in which an ion-selective film having target-ion selectivity was stacked on a base electrode was used. In the present example, the target ion of the ion-selective electrode was set to be Na$^+$.

Regarding the base electrode, efforts to use Ag/AgCl, carbon, and PEDOT/PASS have been proposed, and there is no particular limitation regarding use in the present invention. It is sufficient that the base electrode is selected in accordance with characteristics, such as cost and performance, necessary for the device. In the present example, a Ag/AgCl electrode was adopted as the base electrode.

Regarding the ion-selective film, commonly used ion-selective films may be used provided that the film has sensitivity to a target ion and having sufficient selectivity for an interfering ion. Examples of the material used for the ion-selective film includes ionophores, such as 12-crown-4-ether having a crown ether structure, anion removers, such as sodium tetraphenylborate (NaTPB), plasticizers, such as NPOE and DOS, polymer agents, such as simple PVCs and copolymers of vinyl chloride and vinyl acetate.

Subsequently, the ion-selective film is produced by mixing appropriate amounts of components, dissolving or dispersing them in THF or cyclohexanone serving as a solvent, applying the resulting liquid by an ink jet method to a base electrode (Ag/AgCl electrode) in which intermediate layers such as NaCl are stacked. In this regard, the application method is not limited to the ink jet method, and it is sufficient that the ion-selective film is stacked on the base electrode after the viscosity of the solution is adjusted in accordance with the printing method, such as a dispenser or screen printing.

Regarding this test, the potential profile between the electrodes is obtained in accordance with the $Na^+$ concentration in the specimen, and it is clear that the results indicating sufficient sensitivity (gradient of the graph) and selectivity are obtained.

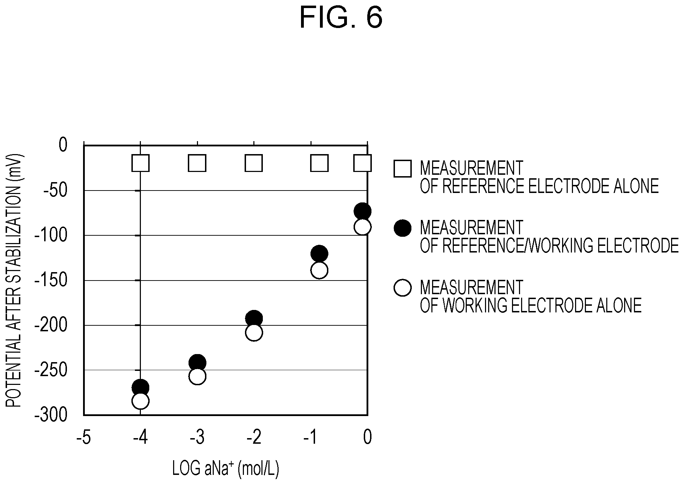

Further, this device was used, a commercially available electrode was set in a dispensation portion between the reference electrode and the working electrode (ion-selective electrode), the potential between the reference electrode and the commercially available electrode was measured (□ plot in Fig. 6), and the potential between the working electrode (ion-selective electrode) and the commercially available electrode was measured (○ plot in Fig. 6).

It is clear that there was substantially no change in the reference electrode-side potential (□) even when the $Na^+$ concentration in the specimen changed and that the ion-selective electrode-side potential (○) changed in accordance with the change in the $Na^+$ concentration. In this regard, the difference of the two is the potential difference between the reference electrode and the working electrode and is indicated by the • plot in Fig. 6, and it was ascertained that the reference electrode exhibited sufficient stability.

In this regard, the present invention is not limited to the micro analysis chip in which the Ag/AgCl electrode is used alone as the reference electrode. As described in NPL 1, a configuration in which Ag/AgCl is used as the base electrode and a support electrolyte layer to stabilize the interfacial potential to the specimen and to decrease the influence of an interfering ion is disposed thereon may be adopted. For example, as described in NPL 1, the support electrolyte layer may be formed by mixing appropriate amounts of TBA-TBB (tetrabutylammonium tetrabutylborate) or TDMACl (tridecylmethylammonium chloride), a plasticizer, and PVC and applying and drying a solution produced by mixing these into THF or cyclohexane serving as a solvent.

<Example 2>

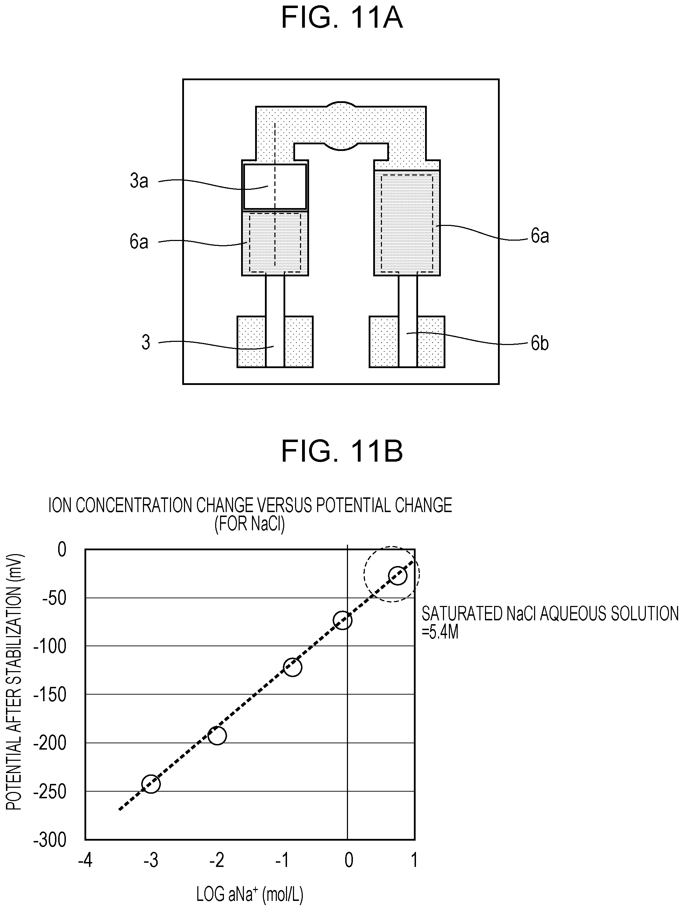

In the present example, as illustrated in Fig. 11A, an ion-selective film 6a is also disposed on the reference electrode. In a considered configuration, an ion-selective film is used where a selected ion is $Na^+$, and NaCl serving as an electrolyte is arranged upstream of the reference electrode. In such an instance, when the specimen dissolves NaCl serving as the electrolyte, in the specimen, $Cl^-$ that reached the saturated concentration and, in addition, $Na^+$ ion that is at the same concentration where the concentration does not change since NaCl is not dissolved any more are present. When the specimen comes into contact with the reference electrode (ion-selective electrode) provided with the ion-selective film, a stable potential is obtained since the $Na^+$ concentration does not change (Fig. 11B).

Regarding this configuration, the electrolyte is not limited to a chloride. When the ion-selective film used for the reference electrode is a $Na^+$-selective film, the electrolyte may be a hydroxide such as sodium hydroxide (NaOH), and the electrolyte may be selected in accordance with the target ion.

<Example 3>

A configuration to make the operation in Example 1 more effective will be described.

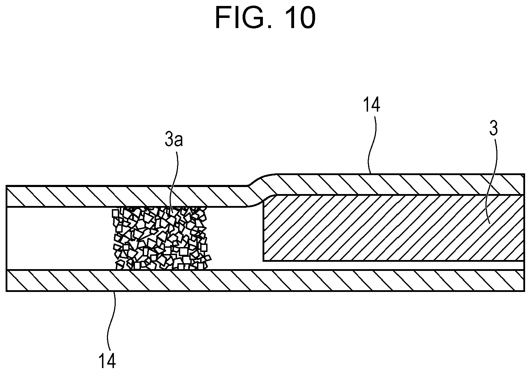

In a configuration in which an electrolyte layer (NaCl or KCl) 3a is arranged upstream of the Ag/AgCl electrode, a laminate layer 14 is disposed on the surface and the back surface of the reference electrode (Fig. 10).

In general, the Ag/AgCl electrode is printed on the paper by screen printing. Since a Ag/AgCl paste ink for screen printing has a certain level of viscosity, the ink is not completely printed in the thickness direction of the filter paper and is printed leaving a certain portion of filter paper on the back surface side. In addition, regarding the flow passage, in consideration of deviation of the Ag/AgCl electrode due to printing, the ink is printed leaving at least about 0.2 mm of clearance from the flow passage wall. Therefore, a filter paper portion functioning as a flow passage remains to the right and left of and under the reference electrode. When the surface of the reference electrode is covered with a laminate layer, a region in which the saturated specimen can move is limited, and the saturated specimen preferentially passes through the filter paper portion remaining to the right and left of and under the reference electrode.

When such a configuration is adopted, only the specimen passed through the limited flow passage comes into contact with the reference electrode, and the specimen in contact with the reference electrode is readily limited to the saturated specimen. Consequently, an output potential is further stabilized.

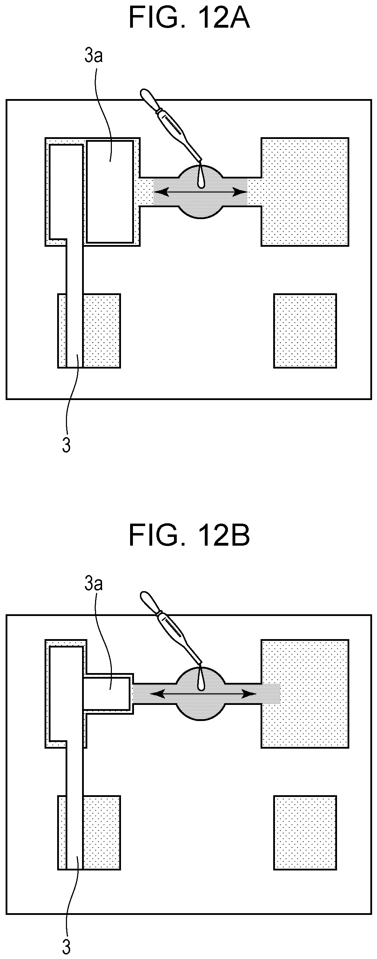

In this regard, the relation between the electrolyte 3a and the reference electrode 3 according to the present invention is not limited to the arrangement configuration illustrated in Fig. 1A to Fig. 1C and may be the arrangement illustrated in Fig. 12A or Fig. 12B. It is sufficient that the electrolyte layer (NaCl or KCl) is arranged upstream of the reference electrode (Ag/AgCl electrode) with reference to a forward direction of the specimen. In addition, even when an area in which the reference electrode is in contact with the specimen is small, a saturated specimen flowing to the reference electrode enables the reference electrode to stably function (Fig. 12B).

The present invention is not limited to the above-described embodiments and can be variously changed and modified without departing from the spirit and scope of the present invention. Therefore, to apprise the public of the scope of the present invention, the following claims are appended.

This application claims the benefit of Japanese Patent Application No. 2022-065895 filed April 12, 2022, which is hereby incorporated by reference herein in its entirety.

**Claims**

1. A micro analysis chip comprising a flow passage region surrounded by a flow passage wall disposed in the interior of a porous substrate,

   wherein the flow passage region includes a first flow passage chamber, a second flow passage chamber, and a flow passage to connect the first flow passage chamber to the second flow passage chamber,
   a reference electrode is disposed in the first flow passage chamber,
   a working electrode is disposed in the second flow passage chamber, and
   an electrolyte is arranged upstream of the reference electrode with reference to a forward direction of a specimen in the flow passage region.

2. The micro analysis chip according to Claim 1, wherein the electrolyte is arranged at a position so that an upstream end portion of the reference electrode is located downstream of a position where an electrolyte concentration in a specimen is a concentration 88% or more of a saturated concentration due to the electrolyte being dissolved in the specimen passing through the flow passage.

3. The micro analysis chip according to Claim 1 or Claim 2, wherein the electrolyte is a chloride.

4. The micro analysis chip according to Claim 3, wherein the chloride is sodium chloride or potassium chloride.

5. The micro analysis chip according to Claim 1 or Claim 2, wherein the electrolyte is a hydroxide.

6. The micro analysis chip according to Claim 4, wherein an amount of the electrolyte is $3.0 \times 10^2$ g/L or more per liter of the specimen supplied to the reference electrode.

7. The micro analysis chip according to Claim 6, wherein an amount of the electrolyte is $3.4 \times 10^2$ g/L or more per liter of the specimen supplied to the reference electrode.

8. The micro analysis chip according to any one of Claims 1 to 7, wherein a base electrode of the reference electrode is a Ag/AgCl electrode.

9. The micro analysis chip according to any one of Claims 1 to 8, wherein the working electrode is an ion-selective electrode in which an ion-selective film is stacked on a base electrode.

10. The micro analysis chip according to any one of Claims 1 to 9, wherein the reference electrode is an ion-selective electrode in which an ion-selective film is stacked on the base electrode.

11. The micro analysis chip according to any one of Claims 1 to 10, wherein a laminate layer is included on a surface of the reference electrode.

## FIG. 1A

## FIG. 1B

## FIG. 1C

# FIG. 2A

# FIG. 2B

# FIG. 3

REFERENCE ELECTRODE CONCENTRATION DEPENDENCE

# FIG. 4

POTENTIAL CHANGE OVER TIME
(NaCl OF 1140 mM OR KCl OF 110 mM IS FIXED)

# FIG. 5A

# FIG. 5B

# FIG. 6

# FIG. 7A

# FIG. 7B

# FIG. 8A

# FIG. 8B

# FIG. 8C

REFERENCE ELECTRODE CONCENTRATION DEPENDENCE

# FIG. 9A

# FIG. 9B

# FIG. 10

# FIG. 11A

# FIG. 11B

ION CONCENTRATION CHANGE VERSUS POTENTIAL CHANGE
(FOR NaCl)

SATURATED NaCl AQUEOUS SOLUTION
=5.4M

FIG. 12A

FIG. 12B

## INTERNATIONAL SEARCH REPORT

| International application No. |
|---|
| **PCT/JP2023/010501** |

### A. CLASSIFICATION OF SUBJECT MATTER

***G01N 27/416***(2006.01)i; ***G01N 27/30***(2006.01)i; ***G01N 35/08***(2006.01)i; ***G01N 37/00***(2006.01)i
FI: G01N27/416 351B; G01N27/30 315Z; G01N35/08 A; G01N37/00 101

According to International Patent Classification (IPC) or to both national classification and IPC

### B. FIELDS SEARCHED

Minimum documentation searched (classification system followed by classification symbols)

G01N27/416; G01N27/30; G01N35/08; G01N37/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JSTChina/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| X | SHITADA, I. et al., Screen-printed Paper-based Three-electrode System with Long-term Stable and Instantaneously Usable Reference Electrode, Chemistry Letters, 2018, vol. 47, pp. 1502-1504, doi:10.1246/cl.180809, and Supporting Information<br>　in particular, abstract, fig. 1, S2 | 1-11 |
| X | SODA, Y. and BAKKER, E., Ionic strength-independent potentiometric cation concentration sensing on paper using a tetrabutylammonium-based reference electrode, Sensors and Actuators: B. Chemical, 01 June 2021, vol. 346, no. 130527, doi.org/10.1016/j.snb.2021.130527<br>　in particular, abstract, p. 2, left column, p. 4, right column to p. 5, left column, scheme 2. | 1-4, 6-10 |
| Y | | 5 |
| X | DING, R. et al., Solid reference electrode integrated with paper-based microfluidics for potentiometric ion sensing, Sensors & Actuators: B. Chemical 323 (2020) 128680, 2020, vol. 323, no. 128680, doi.org/10.1016/j.snb.2020.128680<br>　in particular, abstract, pp. 2-4, fig. 2, 3 | 1–4, 6–9 |
| Y | | 5 |

☑ Further documents are listed in the continuation of Box C.    ☑ See patent family annex.

| | | |
|---|---|---|
| \* | Special categories of cited documents: | |
| "A" | document defining the general state of the art which is not considered to be of particular relevance | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **15 May 2023** | **30 May 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT

| International application No. |
| --- |
| **PCT/JP2023/010501** |

### C.    DOCUMENTS CONSIDERED TO BE RELEVANT

| Category* | Citation of document, with indication, where appropriate, of the relevant passages | Relevant to claim No. |
| --- | --- | --- |
| Y | RUECHA, N. et al., Fully Inkjet-Printed Paper-Based Potentiometric Ion-Sensing Devices, Analytical Chemistry, 2017<br>    in particular, scheme 1. | 1-11 |
| Y | JP 9-178690 A (NGK SPARK PLUG CO., LTD.) 11 July 1997 (1997-07-11)<br>    claims, paragraphs [0017]-[0019], [0026]-[0037], fig. 1-15 | 1-11 |
| Y | JP 2015-514996 A (LUOXIS DIAGNOSTICS, INC.) 21 May 2015 (2015-05-21)<br>    claims, paragraphs [0017], [0031]-[0053], fig. 2-6 | 1-11 |
| Y | JP 2010-14422 A (JAPAN ADVANCED INSTITUTE OF SCIENCE AND TECHNOLOGY) 21 January 2010 (2010-01-21)<br>    claims, paragraphs [0027]-[0036], fig. 3-5 | 1-11 |
| A | US 2016/0033438 A1 (PRESIDENT AND FELLOWS OF HARVARD COLLEGE) 04 February 2016 (2016-02-04)<br>    entire text, all drawings | 1-11 |
| A | US 2012/0181184 A1 (WHITESIDES, George M.) 19 July 2012 (2012-07-19)<br>    entire text, all drawings | 1-11 |
| A | JP 2021-37612 A (CANON INC.) 11 March 2021 (2021-03-11)<br>    entire text, all drawings | 1-11 |
| A | KRIKSTOLAITYTE, V. et al., Paper as sampling substrates and all-integrating platforms in potentiometric ion determination, Trends in Analytical Chemistry, 2020, vol. 133, no. 116070, doi.org/10.1016/j.trac.2020.116070<br>    entire text, all drawings | 1-11 |

Form PCT/ISA/210 (second sheet) (January 2015)

INTERNATIONAL SEARCH REPORT
Information on patent family members

International application No.

**PCT/JP2023/010501**

| Patent document cited in search report | | | Publication date (day/month/year) | Patent family member(s) | | | Publication date (day/month/year) |
|---|---|---|---|---|---|---|---|
| JP | 9-178690 | A | 11 July 1997 | (Family: none) | | | |
| JP | 2015-514996 | A | 21 May 2015 | US 2013/0277232 A1 claims, paragraphs [0015], [0036]-[0059], fig. 2-6 WO 2013/158985 A1 CN 104321645 A KR 10-2015-0013146 A | | | |
| JP | 2010-14422 | A | 21 January 2010 | (Family: none) | | | |
| US | 2016/0033438 | A1 | 04 February 2016 | WO 2014/149611 A1 entire text, all drawings | | | |
| US | 2012/0181184 | A1 | 19 July 2012 | WO 2010/102279 A1 entire text, all drawings | | | |
| JP | 2021-37612 | A | 11 March 2021 | US 2022/0177300 A1 entire text, all drawings WO 2021/039740 A1 EP 4023589 A1 CN 114340776 A | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 62039758 A
- JP 2021037612 A
- JP 2022065895 A

**Non-patent literature cited in the description**

- **NIPAPAN RUECHA** ; **ORAWON CHAILAPAKUL** ; **KOJI SUZUKI** ; **DANIEL CHITTERIO**. Fully Inkjet-Printed Paper-Based Potentiometric Ion-Sensing Devices. *Analytical chemistry*, 29 August 2017, vol. 89, 10608-10616